# EUROPEAN PATENT SPECIFICATION

(11) **EP 2 332 899 B1**
(45) Date of publication and mention of the grant of the patent: **20.03.2013**
(21) Application number: 10164874.9
(22) Date of filing: 03.06.2010
(51) Int. Cl.: C07C 51/235, C07C 59/10

(54) **The method for selective production of glyceric acid**
Das Verfahren zur selektiven Herstellung von Glycerinsäure
Le procédé de production sélective de l'acide glycérique

(30) Priority: 10.12.2009 LV 090217
(43) Date of publication of application: 15.06.2011
(73) Proprietor: Rigas Tehniska universitate, Riga 1658 (LV)
(72) Inventor: Cornaja, Svetlana M, LV-1015, Riga (LV); Kulikova, Lidija, LV-1013, Riga (LV); Serga, Vera, LV-1005, Riga (LV); Kampars, Valdis, LV-1082, Riga (LV); Dubencovs, Konstantins, LV-1046, Riga (LV); Zizkuna, Svetlana M, LV-1006, Riga (LV); Muravjova, Olga, LV-1015, Riga (LV)
(74) Representative: Fortuna, Aleksandra

(56) References cited:
- DATABASE CA [Online] CHEMICAL ABSTRACTS SERVICE, COLUMBUS, OHIO, US; CORNAJA, SVETLANA ET AL: "Liquid-phase catalytic oxidation of glycerol by molecular oxygen in presence of heterogeneous catalysts", XP002624822, retrieved from STN Database accession no. 2008:1469739 & RIGAS TEHNISKAS UNIVERSITATES ZINATNISKIE RAKSTI, SERIJA 1: MATERIALZINATNE UN LIETISKA KIMIJA , 16, 131-141 CODEN: RTUZAL; ISSN: 1407-7353, 2008,
- DATABASE CA [Online] CHEMICAL ABSTRACTS SERVICE, COLUMBUS, OHIO, US; MURAVJOVA, OLGA ET AL: "Glycerol oxidation by molecular oxygen in the presence of a commercial 3% Pd/Al2O3 catalyst in bubble column reactors", XP002624823, retrieved from STN Database accession no. 2010:1324655 & RIGAS TEHNISKAS UNIVERSITATES ZINATNISKIE RAKSTI, SERIJA 1: MATERIALZINATNE UN LIETISKA KIMIJA , 20, 33-42 CODEN: RTUZAL; ISSN: 1407-7353, 2009,

## Description

### Technical Field

The invention relates to chemical technology and namely to selective oxidation of glycerol by molecular oxygen in presence of various supported palladium-containing catalysts.

### Background Art

Glycerol is the main by-product of biodiesel fuel manufacturing; its yield makes up about 14 weight % of overall mass of the products. Glycerol liquid phase catalytic oxidation by molecular oxygen is one of the possible methods of glycerol utilization. Liquid-phase catalytic oxidation makes it possible to conduct the process at mild conditions (at low temperatures and at low oxidizer pressure) using ecologically clean oxidizer (oxygen) and the solvent (water). The use of heterogeneous catalysts has several advantages from the technological point of view: simple process for catalyst regeneration and possibility of its reuse. A number of important and valuable products used as raw products in various processes [1-3] of organic synthesis can be obtained in the process for glycerol oxidation. Currently market of these chemical substances is limited because of their great expenses. The use of process for catalytic oxidation of glycerol by molecular oxygen with the possibility of catalyst regeneration can substantially decrease the prime cost of oxidation products. Platinum group metals are classical supported heterogeneous catalysts [4, 5, 6] used in liquid-phase oxidation process of the alcohols' and aldehydes', including glycerol. During last years great attention has been paid to the use of gold based heterogeneous catalysts in the process for glycerol oxidation [7, 8]. Carbon based carriers (activated carbon, graphite, carbon black) are usually used in the synthesis of supported Pt and Pd catalysts; the metal oxide based carriers are used less frequently. Despite the variety of supported Pd, Pt and Au based catalysts, glycerol oxidation products are mostly obtained in a mixture. Majority of investigations of glycerol oxidation by molecular oxygen are devoted to the development of methods of active and at the same time selective catalyst synthesis [4, 6, 9]. The methods of synthesis of supported Pt catalysts and their activity are still being researched. By varying methods of synthesis of catalysts, catalyst concentration, oxidate pH and temperature, as well as partial pressure of oxygen, the yield and selectivity of glycerol oxidation process products can be changed. Different methods of synthesis of Pd containing catalysts are used when performing catalytic glycerol oxidation: metal sol immobilization method (metal sol fixing on the carrier's surface) [6, 9], impregnation method (carrier impregnation with a metal salt aqueous solution, followed by its reduction) [4, 10].

Various types of reactors are used for glycerol oxidation: closed type batch reactors [10], continuous flow reactors [5] and barbotage reactors with additional stirring of oxidate [4]. The best results in glycerol oxidation by molecular oxygen in the presence of Pd-containing catalyst were achieved in the work [4], where glycerol is oxidized in the presence of 5%Pd/C catalyst. The catalyst was synthesized by the impregnation method from inorganic precursor. Activated charcoal is impregnated by acidic PdCl₄²⁻ ion aqueous solution at 298 K temperature. After impregnation of carrier metal is being reduced by 37% formaldehyde aqueous solution in alkaline medium. After reduction the catalyst is being dried in vacuum at temperature 333 K. The glycerol alkaline water solution is being oxidised in the thermostated barbotage-type glass reactor with stirring at atmospheric pressure and temperature 333 K. The mixer with the stirring speed 1200 rpm is used to stir the oxidate. The oxidation time is 4-8 hours depending on pH of the solution. The best results of glycerol oxidation by molecular oxygen in the presence of 5%Pd/C catalyst disclosed in the scientific article [4] are presented in the table 1.

**Table 1**

| Catalytic oxidation of glycerol by the air molecular oxygen in the presence of 5%Pd/C: C₀(glycerol)= 1 mol/L; n(glycerol/n(Pd)= 300-500 mol/mol | | | | |
|---|---|---|---|---|
| pH | Oxidation time, hours | Rate of glycerol conversion, % | Glyceric acid | |
| | | | Yield, % | Selectivity, % |
| 11 | 4 | 100 | 70 | 70 |
| 11 | 3 | 90 | 70 | 77 |
| 9 | 9 8 | 75 | 41 | 55 |
| 7 | 7 8 | 53 | 16 | 30 |

It can be seen from the table 1 that the glycerol oxidation rate and oxidation time are both depending on the pH of the solution. The increase of solution's pH from 7 to 11 increases glycerol oxidation rate from 53 to 90%, at the same time increases the yield of glyceric acid (from 16 to 70%) and its selectivity (from 30 to 77%). The increase of oxidation rate of glycerol up to 100%, when pH=11, leads to the decrease of glyceric acid's selectivity up to 70%, as a result of glyceric acid's further conversion to oxalic acid (by-product). The significant drawback of the known technical solution is necessity to prepare particularly pure reagent catalyst precursor - acidic PdCl₄²⁻ ion aqueous solution and the reducer - 37% formaldehyde aqueous solution; hereto the expensive equipment is needed for the drying of catalyst in vacuum. It should be mentioned as well, that authors of the article [10] failed to repeat the results received in the article [4]. 5%Pd/C synthesized using the method described in [4] did not show catalytic activity when glycerol was oxidized in similar conditions [10].

There is known the powder-type catalyst 3%Pd/Al₂O₃ (carrier Ba 2/22, the specific surface of the catalyst Sₛₛ= 10 m²/g, the diameter of particles 10 - 90 µm, the specific surface of palladium Sₛₛ= 4,3 m²/g; the producer - W.C. Heraeus GMBH & Co. KG) used for hydrogenation of organic substances. Using this known catalyst the inventors performed experiments for preparation of glyceric acid, described in articles [14, 15]. Using this commercial 3%Pd/Al₂O₃ catalyst for oxidation of glycerol in barbotage-type reactor at 323-333 K temperature the following results were obtained: the maximum yield of glyceric acid 66 - 73% was achieved at glycerol conversion rate 75 - 89 %. The selectivity of the glyceric acid yield ranged from 78 to 92 %, after the glyceric acid yield amounts maximum, its rapid oxidation to the process' by-products started and, by increase of the glycerol conversion rate up to 100 %, the glyceric acid yield and its selectivity sharply decreased. For instance, oxidizing glycerol under the following conditions: the initial molar concentration of glycerol c₀(glyc.) = 0,3 M; the initial molar concentration of sodium hydroxide c₀(NaOH) = 1,5 M; ratio of amount of glycerol and palladium n(glyc.)/n(Pd) = 300 mol/mol; P_{O2} =1,00 atm and 333 K temperature, after the glyceric acid yield amounts maximum (66% at 78% of selectivity), its yield and selectivity decreases up to 35 % and 43 % respectively.

### Disclosure of Invention

The aim of the invention is to increase the yield and selectivity of glyceric acid, as well as make its production more economical. The aim is achieved by oxidation of glycerol by molecular oxygen in alkaline aqueous solutions in the presence of different new supported palladium catalysts and by synthesizing the catalysts using the offered extraction-pyrolytic process.

The offered method for selective production of glyceric acid comprises the following consecutive steps:
(i) obtaining of catalyst metal - palladium containing organic precursor by liquid extraction method, including: adding tetrachloride palladium acid solution in hydrochloric acid to trioctylamine ((C₈H₁₇)₃N) solution in toluene, shaking the mixture obtained, separation the organic phase from the inorganic one and filtering; (ii) adding the carrier, being selected from the group of nanopowders synthesized in plasma, containing: Al₂O₃, Si₃N₄, Y₂O_{3,} to the palladium catalyst organic precursor obtained; (iii) after the impregnation of the carrier by the precursor - drying of the obtained mixture; (iv) pyrolytic reduction of palladium by heating dry carrier up to 573 K at the speed of 10-11 K/min and calcination at 573-673 K for 4-6 minutes at atmospheric pressure; (v) catalytic oxidizing of glycerol by molecular oxygen.

The supported palladium catalysts differ from the prior art solutions by the percentage of palladium composition and by the nature of the carriers as well. Synthesized in plasma Al₂O₃, Si₃N₄ and Y₂O₃ nanopowders with the specific surface Sₛₛ= 12,8 - 46,7 m²/g (depending on the nature of the carrier), are used as carriers according to the invention. The process for preparation of the nanopowders is set forth in scientific article by J. Grabis "Nanosized powders of refractory compounds produced by ICP technique" [11]. In accordance with the proposed invention, nanoporous micro granules being obtained from Al₂O₃ nanopowder can also be used as carriers. Al₂O₃ nanoporous micro granules are being selected having average diameter 100 µm, average diameter of pores in granules - 100 nm, porousness 39 - 40 % and the specific surface Sₛₛ= 27 - 38 m²/g. The process for synthesis of the catalyst starts with production of the organic precursor by liquid extraction method. In order to obtain palladium containing organic precursor by liquid extraction method, trioctylamine ((C₈H₁₇)₃N) solution in toluene is used. (C₈H₁₇)₃N is tertiary amine faintly soluble in water and well soluble in organic solvents. Tetrachloride palladium acid solution in hydrochloric acid (for instance, 2 M hydrochloric acid solution) is being added to the trioctylamine solution in toluene. After shaking of mixture during 3-8 minutes the organic phase is being separated from the inorganic one and filtered. Extraction of palladium from hydrochloric acid aqueous solution by the means of trioctylamine solution in toluene occurs in accordance with the mechanism of anions exchange, forming a compound [(C₈H₁₇)₃NH]₂PdCl₄. For the preparation of precursor it is not necessary to use particularly pure raw materials. Palladium purification from impurity at the step of extraction is being performed. The carrier is being added to the obtained catalyst organic precursor. After the impregnation of carrier by precursor the obtained mixture is being dried for 25 - 35 minutes at 353 - 373 K temperature. Chemical reducers are not necessary for reduction of palladium - palladium is reduced by processing the dry mixture of carrier and precursor by pyrolysis. The dry mixture of carrier is being heated up to 573 K at the speed of 10-11 K/min and calcinated at 573 - 673 K temperature for 4-6 minutes at atmospheric pressure. After the pyrolytic reduction the additional purification of catalyst from admixture is not necessary, because pure catalyst is obtained as a result of reduction. The obtained supported palladium catalysts have highly dispersed palladium particles; content of palladium in catalyst is within the range between 1 and 10 %. The examples of catalysts obtained and their characteristics are given in table 2.

**Table 2**

| Examples of the supported catalysts obtained | | | | |
|---|---|---|---|---|
| Description of Carriers | | Description of Catalysts | | |
| | | Composition | Sₛₛ, m²/g | dₖᵣᵢₛₜ^{*}, nm |
| Al₂O₃ | nanopowder Sₛₛ = 44,2 - 46,7 m²/g | 1 % Pd/ Al₂O₃ | 47,8 | 15 |
| Al₂O₃ | nanopowder Sₛₛ = 44,2 - 46,7 m²/g | 2,5 % Pd/ Al₂O₃ | 36,2 | ∼20 |
| Al₂O₃ | nanopowder Sₛₛ = 44,2 - 46,7 m²/g | 5 % Pd/ Al₂O₃ | 39,5-48,9 | ∼16 |
| A1₂O₃ | nanopowder Sₛₛ = 44,2 - 46,7 m²/g | 1 % Pd/ Al₂O₃ | 39,8 | 20 |
| Al₂O₃ | nanopowder Sₛₛ = 44,2 - 46,7 m²/g | 5 % Pd/ Al₂O₃ | 37,1 | 35 |
| Y₂O₃ | nanopowder Sₛₛ = 20,0 m²/g | 5 % Pd/ Y₂O₃ | 18,6 | 12 |
| Si₃N₄ | nanopowder | 2,5 % Pd/ Si₃N₄ | - | 30 |

| | | | | |
|---|---|---|---|---|
| dₖᵣᵢₛₜ - average size of palladium crystallite; Sₛₛ - the specific surface of catalyst | | | | |

Catalytic oxidation of glycerol can be performed by molecular oxygen at atmospheric pressure in a barbotage type reactor without additional stirring or in closed type batch reactor with magnetic mixer, as well in autoclave at high pressure from 2 to 10 bar (within the range of temperature 328-338 K). The selectivity of glyceric acid production is within the range of 64-85 % at glycerol conversion 69-100 % depending on composition of catalyst and oxidation conditions (table 3). The supported palladium catalysts obtained by the offered method are active in all the examined conditions.

**Table 3**

| Glycerol catalytic oxidation by molecular oxygen in presence of new supported Pd catalysts | | | | | | | | | |
|---|---|---|---|---|---|---|---|---|---|
| No | Catalyst | c₀(glyc) M | c₀(NaOH) M | n(glyc)/ n(Pd) mol/mol | P_{O2,} bar | t, h | Glycerol conversion, % | Glyceric acid | |
| | | | | | | | | Yield % | Selectivity, % |
| 1^{b} | 1,25% Pd/ Al₂O₃ | 0,30 | 0,7 | 400 | 1 | 7,5 | 100 | 76 | 76 |
| 2^{b} | 1,25% Pd/ Al₂O₃ | 0,30 | 1,5 | 400 | 1 | 7,5 | 92 | 68 | 74 |
| 3^{c} | 1,25% Pd/ Al₂O₃ | 0,30 | 1,5 | 300 | 1 | 7,0 | 95 | 70 | 74 |
| 4^{a} | 1,25% Pd/ Al₂O₃ | 0,30 | 1,5 | 400 | 10 | 3,5 | 69 | 44 | 64 |
| 5^{b} | 2,5% Pd/ Al₂O₃ | 0,34 | 1,5 | 458 | 1 | 10,0 | 100 | 72 | 72 |
| 6^{b} | 2,5% Pd/ Al₂O₃ | 0,30 | 0,7 | 300 | 1 | 10,0 | 100 | 78 | 78 |
| 7^{b} | 2,5% Pd/ Al₂O₃ | 0,30 | 0,7 | 400 | 1 | 10,0 | 93 | 72 | 77 |
| 8^{b} | 2,5 % Pd/ Si₃N₄ | 0,30 | 1,5 | 300 | 1 | 7,0 | 19 | 15 | 79 |
| 9^{c} | 5% Pd/ Y₂O₃ | 0,30 | 1,5 | 300 | 1 | 5,0 | 15 | 13 | 87 |

| | | | | | | | | | |
|---|---|---|---|---|---|---|---|---|---|
| T=333 K, a - autoclave, b - barbotage device, c - closed type batch reactor | | | | | | | | | |

Using the offered method, the yield and selectivity of glyceric acid are equal or higher than if glycerol is oxidated in presence of 5 % Pd/C (Tables 1 and 3). Hereto, purification of palladium from impurity is performed during extraction-pirolytic synthesis of catalysts at extraction step. As a result, raw materials of high purity are not necessary for the preparation of the tetrachloride palladium acid aqueous solution, besides reiterative use of palladium containing technological solutions and recycled raw materials is possible. Comparing preparation of glyceric acid by the proposed method with the results of use of the known catalyst 3%Pd/A1₂O₃ [14] it was stated, that in presence of new catalysts at the high conversion of glycerol, glyceric acid could be prepared with greater yield and selectivity.

Oxidation of glycerol by molecular oxygen in presence of supported palladium catalysts in alkaline medium can be performed in thermostated barbotage reactors or closed type devices of periodical operating at atmospheric pressure, as well as in autoclave at Pₒ₂ = 2-10 bar. Glycerol oxidation conditions are the following: T=328-338 K; glycerol initial molar concentration c₀(glyc)=0,3-1,0 M, sodium hydroxide initial molar concentration c₀(NaOH)≥ 0,7 M, ratio of amount of glycerol and palladium n(glyc)/n(Pd)=300-500 mol/mol. Time of glycerol oxidation to glyceric acid depending on the conditions of the process is 3-10 hours (Table 3). For better understanding of the invention, the examples of catalyst preparation and glyceric acid preparation are offered.

### Example 1

Preparation of catalyst precursor. 20 mL 1,0 M (after palladium) tetrachloride palladium acid H₂PdCl₄ solution in 2 M hydrochloric acid is being added to 50 mL of 1M trioctylamine solution in toluene. After shaking of mixture during 5 minutes, the organic phase is being separated from the inorganic one and filtered through the paper filter. The palladium compound [(C₈H₁₇)NH]₂PdCl₄ is being created during shaking of organic precursor in solution. The yield of precursor is 50 mL, concentration of precursor is 0,4 M (after palladium).

### Example 2

Synthesis of 2,5 % Pd/Al₂O₃ catalyst. 2,0 g carrier Al₂O₃ nanopowder is being added to 1,2 mL of precursor solution. The system obtained is being stirred up to carrier impregnation by precursor; then it is being dried for 30 min at 353-373 K temperature. After drying the dry mixture is being heated up to 573 K at the speed 10-11 K/min and calcinated at 573 K temperature for 5 minutes at the atmospheric pressure. Pure 2,5% Pd/Al₂O₃ catalyst is being obtained with specific surface Sₛₛ= 28 m²/g and average size of palladium crystallite d_{cryst}= 15 nm. The yield of 2,5% Pd/Al₂O₃ catalyst is 2,0 g.

### Example 3

The process for glycerol oxidation in barbotage type reactor. Conditions of oxidation: P_{O2}= 1 atm; T = 333 K; c₀(glyc) = 0,3 M; c₀(NaOH) = 0,7 M; n(glyc)/n(Pd) = 300 mol/mol. The total volume of the reactor is 50 mL, the total volume of oxidate is 15 mL. The oxidation is being performed as follows: during thermostating (10 min) 0,0479 g of dry 2,5% Pd/Al₂O₃ catalyst, 11,4 mL distilled water and 1,5 mL 3,0 M glycerol aqueous solution are being fed to the reactor. The supply of oxygen to reactor is being switched on and 2,1 mL 5,0 M sodium hydroxide water solution is being added. The oxidation process starts at the moment of addition of sodium hydroxide. The speed of oxygen flow is 300 mL/min. In order to define the concentration of products of reaction, samples are taken every 60 minutes. Samples of oxydates are being filtered for separation of catalysts. The filtered sample is being acidized by 9,0 M sulfuric acid solution up to pH = 2-3 and analyzed by high performance liquid chromatographic (HPLC) method. After 10 hours glycerol conversion is 100%; selectivity of glyceric acid production - 78 %. The selectivity of by-products production is: tartronic acid - 13 %, glycerolic acid - 5 % and oxalic acid - 4%.

### Example 4

Glycerol oxidation process in a closed type batch reactor with magnetic mixer and gas burette with levelling vessel. The oxidation conditions: Pₒ₂ = 1 atm; T = 333 K; c₀(glyc) = 0,3 M; c₀(NaOH) = 1,5 M; n(glyc)/n(Pd) = 300 mol/mol. The total volume of the reactor is 50 mL, total volume of oxidate is 3 mL. The oxidation experiment is performed as follows: 0,0255 g of dry 1,25% Pd/Al₂O₃ catalyst is being put into the reactor, 1,65 mL distilled water and 0,28 mL 3,2 M glycerol water solution are being introduced. Reactor with reagents and gas burette is being blown and filled with oxygen, after that the reactor is being locked and thermostated for 10 minutes. After thermostating 1,07 mL 4,2 M NaOH solution is being added into the reactor, the magnetic mixer is being switched on and the atmospheric pressure is being set in the system. The speed of mixer spinning is 2500 rpm. Oxidation starts after the mixer is switched on. In order to define the concentration of the reaction products and separate the catalyst, the samples of oxidate are being filtered. The analysis procedure of the reaction products is analogous to the one described in the example 3. After 7 hours the conversion of glycerol is 95 %, selectivity of glyceric acid production - 74 %. The selectivity of by-products production is: tartronic acid - 13 %, glycerolic acid - 7 % and oxalic acid - 3%.

### Example 5

Glycerol oxidation process in autoclave (ROTH, Model II) being connected with a gas burette. The oxidation conditions: P_{O2}= 10 bar; T = 333 K; c₀(glyc) = 0,3 M; c₀(NaOH) = 1,5 M; n(glyc)/n(Pd) = 400 mol/mol. The total volume of the reactor is 200 mL, total volume of oxidate is 30 mL. The experiment of oxidation is being performed as follows: 24,0 mL distilled water, 1,5 mL 3,0 M glycerol water solution and 4,5 mL 5,0 M NaOH solution are is being fed into the reactor. Reactor with reagents and gas burette is being blown, filled with oxygen and thermostated for 30 minutes. After thermostating 0,1916 g dry 1,25% Pd/Al₂O₃ catalyst is being fed into the reactor, oxygen pressure P_{O2} = 10 atm is being set in the system and magnetic mixer is being switched on. The speed of mixer spinning is 1100 rpm. Oxidation starts after the mixer is switched on. In order to define the concentration of the reaction products, samples are being taken every 15 minutes. Samples of oxidate are being filtered in order to separate the catalyst. The analysis procedure of the reaction products is analogous to the one described in the example 3. After 3,5 hours the conversion of glycerol is 69 %, selectivity of glyceric acid production - 64 %. The selectivity of by-products production is: tartronic acid - 19 %, glycerolic acid - 9 % and oxalic acid - 9%.

The proposed invention can be used for the industrial preparation of valuable glycerol oxidation product - glyceric acid. The process for the production is economically beneficial and ecologically clean. At the same time glycerol - the by-product of biodiesel fuel manufacturing is being utilized. The invention provides glyceric acid production by catalytic oxidation of glycerol by molecular oxygen in alkaline water solutions in presence of various supported palladium catalysts. Glyceric acid is being used as raw material for manufacturing of cosmetics [1,2], biodegradable surfactants and biodegradable polymers [3,12,13].

### Sources of information

[1] Patent US 6630130, published 07.10.2003, (Grimes Pearl et. al.).
[2] Patent JP 4120008, published 21.04.1992, (Kojima Hatsuo et. al.).
[3] E.Chiellini. Polyesters Based on Glyceric Acid Derivatives as Potential Biodegradable Materials. J. Bioact. Compat. Polym., 1990, 5(1), 16 - 30.
[4] R.Garcia, M.Besson. P.Gallezot. Chemoselective catalytic oxidation of glycerol with air on platinum metals. Applied Catalysis: A, 1995, 127, 165-176.
[5] P.Fordham, R.Garcia, M.Besson, P.Gallezot. Selective catalytic oxidation with air of glycerol and oxygenated derivatives on platinum metals. Stud. Surf. Sci. Catal., 1996, 101, 161 - 170.
[6] C.L.Bianchi, P.Canton, N.Dimitratos, F.Porta, L.Prati. Selective oxidation of glycerol with oxygen using mono and bimetallic catalysts based on Au, Pd and Pt metals. Catalysis Today, 2005, 102-103, 203-212.
[7] A.S.K. Hashmi, G.J.Hutchings. Gold Catalysis. Angew. Chem. Int. Ed., 2006, 45, 7896 - 7936.
[8] Patent LV 13956 "Gliceri̅nska̅bes iegu̅ 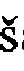 anas metode un katalizators ta̅s realiza̅cijai", published 20.11.2009, (S. 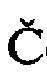 ornaja, V. Kampars, J. Grabis, D. Jankovi 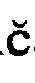 a, O. Muravjova, S. 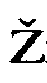 i 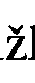 kuna, R. Kampare, K. Dubencovs).
[9] N.Dimitratos, F.Porta, L.Prati. Au, Pd (mono and bimetallic) catalysts supported on graphite using the immobilisation method. Synthesis and catalytic testing for liquid phase oxidation of glycerol. Applied Catalysis: A, 2005, 291(1-2), 210-214.
[10] S.Carrettin, P.McMorn, P.Johnston, K.Griffin, C.J.Kiely, G.J.Hutchings. Oxidation of glycerol using supported Pt, Pd and Au catalysts. Phys. Chem. Chem. Phys., 2003, 5, 1329 - 1336
[11] J. Grabis. Nanosized powders of refractory compounds prodused by ICP technique. Latvijas Kimijas 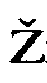 urna̅ls, 2003, 4, 316 - 326.
[12] Patent US 5500139, published 19.03.1996 (Rahman Mohammad A et. al.).
[13] Patent JP 8104892, published 23.04.1996 (Hayashi Hiromitsu et. al.).
[14] O. Muravjova, K. Dubencovs, S. 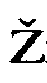 i 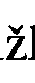 kuna, V. Kampars, S. 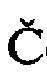 ornaja. Gliceri̅na okside̅ 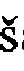 ana ar molekula̅ro ska̅bekli komercia̅la 3%Pd/Al₂O₃ katalizatora kla̅tbu̅tne̅ barbota̅ 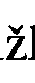 as tipa reaktoros.// RTU Zina̅tniskie raksti, se̅rija Materia̅lzina̅tne un lieti 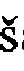 ka̅ 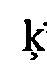 i̅mija, 2009., 20. se̅jums, 33-42.
[15] S. 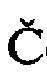 ornaja, S. 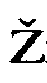 i 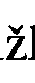 kuna, V.Kampars, K.Dubencovs, O.Muravjova. Liquid-phase catalytic oxidation of glycerol by molecular oxygen in presence of heterogeneous catalysts.// RTU Zinatniskie raksti, volume 16, 2008, pages 131-141.

## Claims

1. A method for selective production of glyceric acid, comprising the following consecutive steps:
(i) obtaining catalyst metal - palladium containing organic precursor by liquid extraction method, comprising:
(a) adding tetrachloride palladium acid solution in hydrochloric acid to trioctylamine ((C₈H₁₇)₃N) solution in toluene;
(b) shaking the mixture obtained;
(c) separation the organic phase from the inorganic one and filtering;
(ii) adding of the carrier, being selected from the group of nanopowders synthesized in plasma, containing: Al₂O₃, Si₃N₄, Y₂O₃ to the palladium catalyst organic precursor obtained;
(iii) drying the obtained mixture after the impregnation of the carrier by the precursor;
(iv) the pyrolytic reducing of palladium by heating of the dry carrier mixture up to 573 K at the speed of 10-11 K/min and calcination at 573-673 K for 4-6 minutes at atmospheric pressure;
(v) the catalytic oxidation of glycerol by molecular oxygen.

2. The method according to claim 1, **characterized in that** at the step of glycerol catalytic oxidation by molecular oxygen, the oxidation of the glycerol is being performed in alkaline water solution at the temperature 328-338 K, the initial molar concentration of glycerol c₀(glyc.)=0,3 -1,0 M; the initial molar concentration of sodium hydroxide c₀(NaOH)≥0,7 M; the ration of amount of glycerol and palladium n(glyc.)/n(Pd)=300-500 mol/mol.

3. The method according to claim 2, **characterized in that** glycerol oxidation is being performed at atmospheric pressure.

4. The method according to claim 2, **characterized in that** glycerol oxidation is being performed at high pressure from 2 to 10 bar.

5. The method according to any of the preceding claims, **characterized in that** the content of palladium in catalyst is within the range between 1 and 10 %.

6. The method according to any preceding claims, **characterized in that** the carrier is nanoporous micro granules, being obtained from Al₂O₃nanopowder.

7. The method according to claim 6, **characterized in that** nanoporous micro granules are being selected having average diameter 100 µm, average diameter of pores in granules 100 nm, porousness 39-40 % and the specific surface Sₛₛ= 27-38 m²/g.

## Patentansprüche

1. Die Methode für die selektive Herstellung von Glycerinsäure umfasst folgende Schritte:
(i) Die Gewinnung von Palladium als Katalysatormetall, der einen organischen Precursor enthält erfolgt anhand einer Flüssigextraktionsmethode und umfasst:
(a) Das Hinzufügen von Tetrachlorkohlenstoff-Palladiumsäurelösung in Salzsäure zu einer Trioctylaminlösung ((C₈H₁₇)₃N) in Toluen;
(b) Schütteln der erhaltenen Mischung;
(c) Trennung der organischen von der inorganischen Phase und Filterung;
(ii) Hinzufügen des Trägers, der aus der Gruppe der in Plasma synthetisierten Nanopulver gewählt wird und Folgendes enthält: Al₂O₃, Si₃N₄, Y₂O₃ zu der erhaltenen Vorstufe des Palladiumkatalysators;
(iii) Trocknung der erhaltenen Mischung nach der Imprägnierung des Trägers durch den Precursor;
(iv) Pyrolytische Reduktion des Palladiums durch Erwärmung der trockenen Trägermischung auf 573 K bei einer Geschwindigkeit von 10 - 11 K/min und einer Kalzinierung bei 573 - 673 K für die Dauer von 4 - 6 Minuten bei Atmosphärendruck;
(v) Katalytische Oxidation des Glycerins durch molekularen Sauerstoff.

2. Die Methode gemäß Anspruch 1 ist **dadurch gekennzeichnet, dass** beim Schritt der katalytischen Oxidation des Glycerins durch molekularen Sauerstoff die Oxidation des Glycerins in einer alkalischen Wasserlösung bei einer Temperatur von 328 - 338 K durchgeführt wird, die anfängliche Molkonzentration des Glycerins c₀(glyc.)=0,3 - 1,0 M beträgt; die anfängliche Molkonzentration des Natriumhydroxids c₀(NaOH)≥0,7 M beträgt; das Verhältnis der Menge von Glycerin und Palladium n(glyc.)/n(Pd)=300-500 mol/mol ist.

3. Die Methode gemäß Anspruch 2 ist **dadurch gekennzeichnet, dass** die Glycerinoxidation unter Atmosphärendruck stattfindet.

4. Die Methode gemäß Anspruch 2 ist **dadurch gekennzeichnet, dass** die Glycerinoxidation bei einem hohen Druck zwischen 2 und 10 bar stattfindet.

5. Die Methode gemäß den vorhergehenden Ansprüchen ist **dadurch gekennzeichnet, dass** der Anteil an Palladium im Katalysator innerhalb eines Bereichs von 1 und 10 % liegt.

6. Die Methode gemäß den vorhergehenden Ansprüchen ist **dadurch gekennzeichnet, dass** die nanoporösen Mikrogranulate aus dem Al₂O₃ Nanopulver gewonnen werden

7. Die Methode gemäß Anspruch 6 ist **dadurch gekennzeichnet, dass** die gewählten nanoporösen Mikrogranulate einen mittleren Durchmesser von 100 µm haben, einen mittleren Porendurchmesser der Granulate von 100 nm, eine Porosität von 39 - 40 % und eine spezielle Oberfläche von Sₛₛ= 27-38 m²/g

## Revendications

1. Procédé de production sélective d'acide glycérique, comprenant les étapes consécutives suivantes :
(i) obtenir un précurseur organique contenant un métal catalyseur/du palladium par procédé d'extraction liquide, comprenant:
(a) ajouter une solution d'acide de tétrachlorure de palladium dans de l'acide chlorhydrique à une solution de trioctylamine ((C₈H₁₇)₃N) dans du toluène ;
(b) agiter le mélange obtenu;
(c) séparer la phase organique de la phase inorganique puis filtrer;
(ii) ajouter le support, sélectionné dans le groupe de nanopoudres synthétisées dans le plasma, contenant: Al₂O₃, Si₃N₄, Y₂O₃ au précurseur organique de catalyseur au palladium obtenu;
(iii) sécher le mélange obtenu après l'imprégnation du support par le précurseur;
(iv) la réduction pyrolytique du palladium par chauffage du mélange sec du support jusqu'à 573 K à une vitesse de 10 à 11 K/min et la calcination à 573 à 673 K pendant 4 à 6 minutes sous pression atmosphérique;
(v) l'oxydation catalytique de glycérol par l'oxygène moléculaire.

2. Procédé selon la revendication 1, **caractérisé en ce que**, à l'étape d'oxydation catalytique de glycérol par l'oxygène moléculaire, l'oxydation du glycérol est réalisée dans une solution alcaline d'eau à la température de 328 à 338 K, la concentration molaire initiale de glycérol c₀(glyc.) = 0,3 à 1,0 M ; la concentration molaire initiale d'hydroxyde de sodium c₀(NaOH) > 0,7 M ; le rapport de quantité de glycérol et de palladium n(glyc.)/n(Pd) = 300 à 500 mol/mol.

3. Procédé selon la revendication 2, **caractérisé en ce que** l'oxydation de glycérol est réalisée sous pression atmosphérique.

4. Procédé selon la revendication 2, **caractérisé en ce que** l'oxydation de glycérol est réalisée à une pression élevée de 2 à 10 bar.

5. Procédé selon l'une quelconque des revendications précédentes, **caractérisé en ce que** la teneur en palladium dans le catalyseur est dans la plage comprise entre environ 1 et 10 %.

6. Procédé selon l'une quelconque des revendications précédentes, **caractérisé en ce que** le support consiste en des micro granules nanoporeux, obtenus à partir de la nanopoudre Al₂O₃.

7. Procédé selon la revendication 6, **caractérisé en ce que** les micro granules nanoporeux sélectionnés ont un diamètre de 100 µm, un diamètre moyen de pores dans les granules de 100 nm, une porosité de 39 à 40 % et une surface spécifiques Sₛₛ= 27 à 38 m²/g.
